# EUROPEAN PATENT APPLICATION

(11) **EP 2 601 897 A1**
(43) Date of publication of application: **12.06.2013**
(21) Application number: 12195134.7
(22) Date of filing: 30.11.2012
(51) Int. Cl.: A61B 17/34

(54) **Access device for cardiac stent delivery and method for fixing the same**

(30) Priority: 07.12.2011 EP 11192297
(71) Applicant: JenaValve Technology Inc., Wilmington, DE 19801 (US)
(72) Inventor: Bauernschmitt, Robert, 81247 München (DE); Straubinger, Helmut, 85609 Aschheim (DE); Litzenburger, Michael, 80333 München (DE)
(74) Representative: Meissner, Bolte & Partner

(57) **Abstract**

The present disclosure relates to an access device (100) for cardiac stent delivery, in particular for delivery of a expandable stent having a replacement heart valve affixed thereto, comprising a tubular element (110) adapted to be sealably inserted into an opening of a wall (500) of the heart for communication with the interior of the heart. In order to provide a device for realization of a sealed and stable passage through the apical area of the heart during beating heart surgery the device comprises at least two radially outwardly extending attachment means (141, 142) adapted to engage with concentric purse-string sutures (900) surrounding the opening of the wall (500) of the heart for fixing the access device (100) to the wall (500) of the heart. Furthermore the present disclosure relates to a method suitable for establishing a stable fixation of the access device (100), such that the risk of a trauma to the tissue of the apex can be minimized.

## Description

The present invention relates generally to an access device for cardiac stent delivery and a method for fixing the same. More specifically, the invention relates to an apical access device deployable during beating heart surgery. The access device according to the present invention may be used for implanting a heart valve stent, in particular an expandable heart valve stent having a prosthetic heart valve affixed thereto, in a minimally invasive way.

Medical technology has long since endeavored to correct valvular defects such as, for example, aortic valve insufficiencies or aortic valve stenosis, without requiring open heart surgery by means of minimally invasive methods. During the last decades minimally invasive forms of treatment have been developed and improved. They are in particular characterized in that a catheter delivery system is employed in order to advance to the site inside the body where, e.g. implantation of a prosthetic device is required. Since by employing a catheter delivery system only small incisions are necessary resulting in a faster patient recovery with less pain and bodily trauma can be achieved. Furthermore, in particular, in the case of performing a minimal invasive heart surgery the patient must not be placed on cardiopulmonary bypass for the duration of the surgery allowing the procedure to be performed under local anesthesia. This, in turn, reduces the medical costs and the overall disruption to the life of the patient.

The term "medical delivery system" as used herein generally refers to a medical system with which a stent system can be advanced in minimally invasive fashion to the site of implantation in the patient's heart, for example to treat an aortic valve stenosis and/or aortic valve insufficiency. In the present context, "minimally invasive" means a heart-lung machine is not needed when performing the procedure on the anaesthetized patient such that the medical procedure can not only be performed at reasonable cost, but there is also less physical and psychological strain on the patient.

The terms "aortic valve stenosis and/or aortic valve insufficiency" as used herein generally refer to a congenital or acquired dysfunction of one or more cardiac valves. Such valvular disorders can affect any of the four cardiac valves, whereby the valves in the left ventricle or left chamber aortic and mitral valve are typically more affected than those on the right side of the heart pulmonary and tricuspid valve. The dysfunction can be a constriction stenosis, an incompetence insufficiency or a combination of the two combined vitium.

In this disclosure, the expression "catheter system" means a system that can be inserted into a body cavity, duct or vessel. A catheter system thereby allows access by surgical instruments. The process of inserting a catheter system is catheterisation. In most uses a catheter system is a thin, flexible tube: a "soft" catheter system; in some uses, it is a larger, solid tube: a "hard" catheter system.

A medical delivery system usually comprises a catheter system by means of which a stent, as needed with a prosthetic heart valve affixed thereto in a folded state, can be introduced into the patient's body in its folded state. For example, the medical delivery system can exhibit a catheter tip having at least one manipulatable receiving area at a proximal end section of the catheter system; i.e. closest to the heart. It is moreover conceivable for the medical delivery system to exhibit a handle at the proximal end section of the catheter system; i.e. at the end section of the catheter system farthest from the heart and the catheter tip, with which the at least one receiving area of the caterer tip can be appropriately manipulated such that the expandable stent accommodated in the catheter tip, as needed with a prosthetic heart valve affixed thereto, can be incrementally released from the catheter tip according to a predefined or predefinable sequence of events.

Generally, there are two minimally invasive approaches known for implanting a prosthetic heart valve. The first approach is the so-called transarterially or transfemorally approach in which, e.g. an expandable heart valve stent with a heart valve prosthesis attached thereto is advanced to the implantation site via the aorta of a patient. The second approach is the so-called transapical approach, wherein access to the heart is provided through the apical area of the heart in order to introduce, e.g. an expandable stent system.

Generally, the apical area or apex of the heart corresponds to the blunt rounded inferior extremity of the heart formed by the left and right ventricles.

After reaching the implantation site with the transapical approach, the stent with the prosthetic heart valve affixed thereto can then be positioned and unfolded. After unfolding, the prosthetic heart valve can be anchored in the desired position in the heart, for example with the aid of anchoring hooks. The actual prosthetic heart valve is usually positioned in the inflow area of the stent.

The use of a minimally invasive transarterial or transfemoral approach, however, introduces some complexities to the surgical procedure. An inherent difficulty in the transarterial or transfemoral approach is the limited space that is available within the vasculature. Here, the surgical field available is only as large as the diameter of a blood vessel. Consequently, the introduction of tools and prosthetic devices presents substantial technical challengers, On the one hand, the prosthetic device must be dimensioned and configured such that introduction into the vasculature, manoeuvring there through, and positioning at a desired location is possible. This may involve passage through considerable convolutions at some distance from the initial point of introduction.

In contrast, with a minimally invasive transapical approach a direct access to the heart through the heart wall, e.g. the apex of the heart, may be provided in a less complicated way. In particular, a minimally invasive transapical approach is not as much limited as by the size constraints which are present in the transfemoral approach. While access to the heart through the femoral vessels in the transfemoral approach is limited to the diameter of the vessel approximately 8 mm, access to the heart through the apical area is significantly larger approximately 25 mm. Additionally, compared to the transfemoral approach, with the transapical approach the distance from the initial point of introduction to the implantation site is shorter. This allows a better controllability during the surgery as well as easier placement and positioning of the prosthetic device.

Thus, apical access to the heart permits greater flexibility with respect to the types of devices and surgical methods that may be performed in the heart. Furthermore, the unique anatomical structure of the apical area permits the introduction of various surgical devices and tools into the heart without significant disruption of the natural mechanical and electrical heart function, enabling surgery without cardiopulmonary bypass of the patient by means of a heart-lung machine.

However, the transapical implantation approach poses problems, in particular associated with respect to the fact that the apical access passage used for the introduction of instruments during surgery needs to be sealed in order prevent significant blood loss and outflow of blood from the ventricle while the heart is pumping. Apart from the problem of sealing, there is also the fundamental problem that the tissue at the apex is quite sensitive and can easily be damaged and is prone to rupture. In conjunction hereto, often likewise regarded as problematic is that the change and manipulation of surgical instruments introduced through the passage of the apex often leads to enhanced stress to the apical area increasing the risk of tissue damage. This is especially due to the fact that during the manipulation of instruments introduced trough the passage in the apex relative motions between the instruments and the wall of the heart occur. In case of beating heart surgery, these relative motions are even further pronounced since the wall of the heart is moving while the heart is pumping. Therefore, the risk of severe tissue damage to the surrounding tissue of the passage in the apex is increased.

Accordingly, it is an object of this invention to provide a device which enables access through the heart wall, preferably the apical area of the heart during beating heart surgery by realization of a sealed passage with an access device according to the invention as disclosed herein, while at the same time minimizing the risk of a trauma to the tissue of the apex by providing a method suitable for establishing stable fixation of the device to the heart, or to provide a solution or improvement of devices and procedures of the state of the art.

On the basis of the problems outlined above, the present disclosure relates to an access device for cardiac stent delivery as well as to a method for fixing the same to the apex of the heart with which the problems are overcome.

Embodiments of the present disclosure may provide an access device for delivery of an expandable stent, as needed with a prosthetic heart valve affixed thereto, into the heart, in particular to an access device which can be fixed to the apex of the heart by appropriate attachment means adapted to engage with concentric purse string sutures surrounding the opening of the wall of the heart.

Preferably, the inventive access device and method for fixing the same provide a tool for simplifying and improving the transapical access for beating heart surgery.

Piercing of the heart surface in order to provide the opening in the wall of the heart for the introduction of the access device may be done prior to inserting the access device.

A preferred embodiment is an access device comprising a tubular element adapted to be sealably inserted into an opening of a wall of the heart for communication with the interior of the heart. The access device is configured, such that communication with the interior of the heart is possible and a catheter can be introduced and inserted into the heart. For fixing the access device to the wall of the heart, said access device includes at least two radially outwardly extending attachment means adapted to engage with concentric purse-string sutures surrounding the opening of the wall of the heart. The at least two radially outwardly extending attachment means are preferably diametrically opposed and may be realized in form of L-shaped and/or T-shaped and/or quadrant-shaped and/or any circle segmental-shaped hooks.

In some embodiments of the access device, the tubular element comprises a side port extending at an angle from the tubular element, wherein the side port is suitable for introduction of medicaments or other fluids through the lumen of the tubular element.

In some embodiments of the device, at least one of the at least two attachment means is rotatable relative to the other diametrically opposed attachment means. Preferably the rotational attachment means is mounted on the access device, such that a rotation around the axis of the access device of about +/-30° is possible.

In some embodiments of the access device, one of the at least two attachment means is an integral part of the tubular element of the access device or fixedly connected to the tubular element of the access device. The other of the at least two attachment means is an integral part of a ring or fixedly connected to a ring, which is rotatable mounted on the exterior surface of the tubular element.

In some embodiments of the access device, the ring is mounted on the exterior surface of the tubular element, such that the ring slides on a surface of one of the at least two attachment means which faces towards a proximal end of the access device.

In some embodiments of the access device, the tubular element of the inventive access device comprises a first portion having a first outer diameter and a second portion having a second outer diameter, wherein the first outer diameter is smaller than the second outer diameter such that a stop is formed having a surface which is substantially perpendicular to the axis of the tubular element. Said surface is adapted to form a sealed contact with the outer surface of the wall of the heart, when the access device is introduced into the opening of the wall. The at least two attachment means are connected to the second portion of the tubular element, having the larger outer diameter.

In some embodiments of the access device, one of the at least two attachment means is an integral part of the second portion of the tubular element or fixedly connected to the second portion of the tubular element of the access device. The other of the at least two attachment means is an integral part of a ring or fixedly connected to a ring, which is rotatable mounted on the exterior surface of the first portion of the tubular element of the access device.

In some embodiments of the access device, the access device comprises two rings with at least one attachment means on each ring, wherein both rings are rotatable mounted on the exterior surface of the tubular element.

In some embodiments of the access device, the access device comprises two rings with at least one attachment means on each ring. At least one of the two rings is rotatable mounted on the exterior surface of the tubular element of the access device, while the other ring is fixedly mounted on the exterior surface of the tubular element by appropriate fixing means, e.g. by a clip mechanism.

In some embodiments of the access device, the at least two rings with the at least attachment means may be mounted on the exterior surface of the second portion of the tubular element of the access device. At least one of said at least two rings may be rotatable around the axis of the access device.

In some embodiments of the access device, the second portion of the tubular element of the access device is a separate part, which is fixedly connected to the first portion of the tubular element by appropriate attachment means, e.g. by a clip mechanism.

In some embodiments of the access device, the access device comprises two tubular elements, wherein a first tubular element having a smaller or equal outer diameter than the inner diameter of the second tubular element is accommodated or can be received in the second tubular element.

In some embodiments of the access device, the access device comprises at least one ring with at least three attachment means, wherein the at least three attachment means are distributed equally around the circumference of the ring, such that the spacing between two neighboring attachment means is equal for all at least three attachment mean.

In some embodiments of the access device, the length of the tubular element of the access device can be adjusted along the axis of the tubular element, e.g. by employing a telescope mechanism.

In some embodiments of the access device, the distal end of the access device comprises a radiopaque tip in order to visualize the position of the access device in the operating field inside the heart by means of an appropriate imaging system. The imaging system may be used at any time or throughout the duration of the surgery.

In some embodiments of the access device, the attachment means can be designed, such that they are in the same or in a different axial plane than the ring itself, to which the attachment means are connected. The design of a ring with attachment means, in which the attachment means are in a different axial plane than the ring, can for example be realized by providing an axially bended transition region from the ring to the attachment means.

In some embodiments of the access device, the second portion of the tubular element of the access device, may be designed to form a ergonomically handle.

The various embodiment of the inventive access device are preferably made of biocompatible material, such as stainless steel, Ti, Ti alloys, Ni alloys, Co alloys and biocompatible polymers.

A preferred method for fixing the access to the wall of the heart, particularly to the apical area of the heart, includes providing an access device according to the invention; inserting the access device into the opening of the wall of the heart; engaging the attachment means with concentric purse-string sutures surrounding the opening of the wall of the heart; feeding the ends of the purse-string sutures through a tourniquet, pulling the ends of purse-string sutures whereby a force is transmitted to the wall of the heart which is directed to the interior of the heart via the attachment means, such that a stable and sealed connection is established between the wall of the heart and the access device; and immobilizing the ends of the purse-string sutures at the proximal end of the tourniquet such that the distal end of the tourniquet is pressed against the wall of the heart and/or against the attachment means, such that the established connection between the wall of the heart and the access device is sustained.

The above aspects, other features and advantages of the present invention are described in greater detail below in connection with drawings of a preferred system and method, which is intended to illustrate, but not to limit the present invention.

Shown are:
- Fig. 1a: a perspective view of the inventive access device for cardiac stent delivery according to a first embodiment, wherein the device comprises a tubular element adapted to be sealably inserted into an opening of a wall of the heart for establishing a passage for communication with the interior of the heart, and wherein the device comprises attachment means adapted to engage with concentric purse-string sutures surrounding the opening of the wall of the heart for fixing the position of the access device;
- Fig. 1b: an axial cross-sectional view of the access device according to Fig. 1a;
- Fig. 1c: a horizontal cross-sectional view of the access device according to Fig. 1a along the line A-A as indicated in Fig. 1a;
- Fig. 1d: a cross-sectional view of the access device according to Fig. 1a inserted into the opening in the apical area of the heart;
- Fig. 2a: a perspective view of the inventive access device for cardiac stent delivery according to a preferred embodiment, wherein one of the two attachment means is fixedly connected to the exterior surface of the tubular access device while the other attachment means is part of a ring which is rotatable mounted on the exterior surface of the access device;
- Fig. 2b: an axial cross-sectional view of the access device according to a preferred embodiment as shown in Fig. 2a;
- Fig. 3a: a perspective view of the inventive access device for cardiac stent delivery according to a prferred embodiment, wherein the access device comprises a first tubular portion and a second tubular portion, wherein the first tubular portion has a smaller outer diameter than the second tubular portion, such that a stop is formed adapted to form contact with the outer surface of the wall of the heart;
- Fig. 3b: an axial cross-sectional view of the access device according to a preferred embodiment as shown in Fig. 3a;
- Fig. 4: a perspective view of the inventive access device for cardiac stent delivery according to a preferred embodiment, wherein one of at least two attachment means is connected to the exterior surface of the second tubular portion of the access device while the other attachment means is part of a ring which is rotatable mounted on the exterior surface of the first tubular portion;
- Fig. 5: a perspective view of the inventive access device for cardiac stent delivery according to a preferred embodiment, wherein the device comprises two rings each with at least one attachment means;
- Fig. 6: an axial cross-sectional view of an access device comprising two ring each with at least one attachment means according to another preferred embodiment;
- Fig. 7: a perspective view of the inventive access device for cardiac stent delivery according to a preferred embodiment, wherein the device comprises a ring with three attachment means;
- Fig. 8a: horizontal cross-sectional view of a ring for visualizing a possible realization of the attachment means, wherein the attachment means is T-shaped;
- Fig. 8b: horizontal cross-sectional view of a ring for visualizing a possible realization of the attachment means, wherein the attachment means has a concentric curved shape;
- Fig. 8c: horizontal cross-sectional view of a ring for visualizing a possible realization of the attachment means, wherein the attachment means has a quadrant- or L-shape;
- Fig. 8d: horizontal cross-sectional view of a ring for visualizing a possible realization of the attachment means, wherein the attachment means are planar with the ring;
- Fig. 8e: horizontal cross-sectional view of a ring for visualizing a possible realization of the attachment means, wherein the attachment means are connected to the ring by a bended transition region, such that the attachment means are in a different axial plane than the ring;
- Fig. 8f: horizontal cross-sectional view of a ring for visualizing a possible realization of the attachment means, wherein the attachment means has a hook-like shape in axial direction; and
- Fig. 9: a perspective view of the inventive access device, wherein the attachment means are engaged with concentric purse-string sutures surrounding the opening of the wall of the heart.

Although, the transapical approach to the heart permits greater flexibility with respect to the types of devices and surgical methods that may be performed in the heart, establishing a sealed passage for communication with the interior of the heart still remains a challenge. Especially, due to the fact that usually the transapical procedure is performed while the heart is beating. Furthermore, the change and manipulation of surgical instruments introduced through the passage of the apex typically leads to enhanced stress in the apical area increasing the risk of tissue damage. Therefore, a need for a device as well as for a method for fixing the same to the heart exists, which are suitable to overcome the problems associated with the transapical minimally invasive approach as outlined above. In this context it will be appreciated that it is within the reach of the invention that the devices and methods of the invention are useful for transapical as well as any opening of a heart or the heart wall for access to the interior of the heart and for the deployment of devices into the heart even though it will be exemplified in the following with regard to transapical surgery.

Certain positions or directions of movement of components of the various embodiments of the access device according to the present invention may be described in relation to heart of the patient. Therefore, the distal end of the access device corresponds to the end which is introduced into an opening in the wall of the heart, while the proximal end of the access device refers to the end which is at the exterior of the heart.

Furthermore, all embodiments of the inventive access device shown in the drawings are only of schematic nature. Hence, dimensions of the access device itself as well as components of the access device can not be deduced from the schematic drawings. However, the figures as disclosed herein are intended to illustrate the various aspects of the present invention.

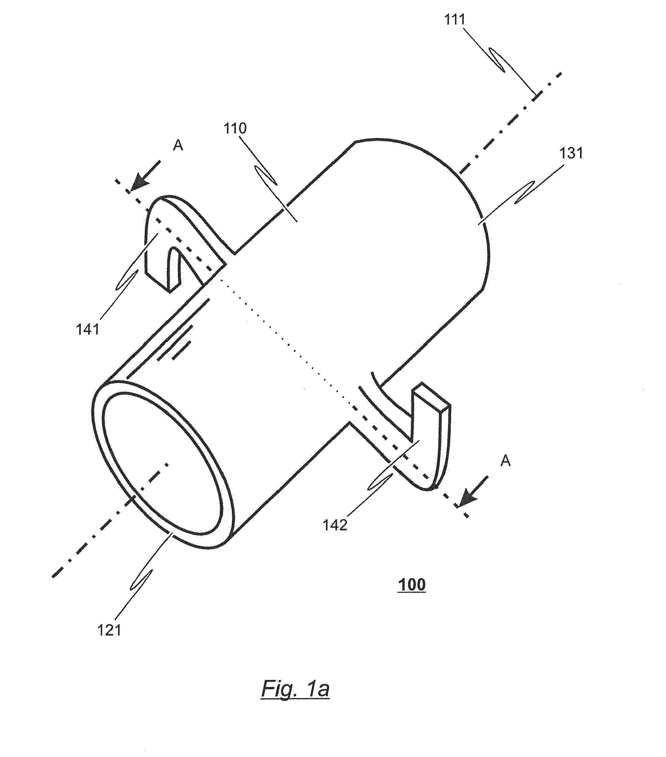

The inventive access device described below for example with reference to Fig. 1a to Fig. 1d is suited to form a passage through the apical area of the heart for the delivery of an expandable stent having a replacement heart valve affixed thereto during beating heart minimally invasive surgery. The access device according to the present invention is adapted to allow a sealed communication with the interior of the heart through an opening of a wall of the heart.

The following description serves to present an example of the design of a access device for the delivery of an expandable stent having a replacement heart valve affixed thereto which suitably designed for a transapical approach, whereby the device aids establishing a sealed passage through the wall of the heart during the surgical procedure.

An exemplary embodiment of the access device 100 according to the invention will be described below referencing Fig. 1a to 1d.

The access device enables an expandable heart valve stent to be implanted transapically in a patient's body; i.e. advanced from the apex of the heart. To this end, the access device 100 comprises a tubular element 110 which is introduced in a opening of the wall 500 of the heart at its apical area. Furthermore, the access device 100 as depicted in Fig. 1a comprises attachment means 141, 142 by means of which the access device 100 can be positioned and fixed to the heart. The attachment means 141, 142 are extending radially outwardly from the tubular element 110 of the access device 100, such that they can be brought into engagement with concentric purse-string sutures 900 surrounding the opening of the wall 500 of the heart. As shown in Fig. 1a to 1c, the attachment means 141, 142 are integrally connected to the tubular element 110 of the access device 100 and diametrically opposed to each other.

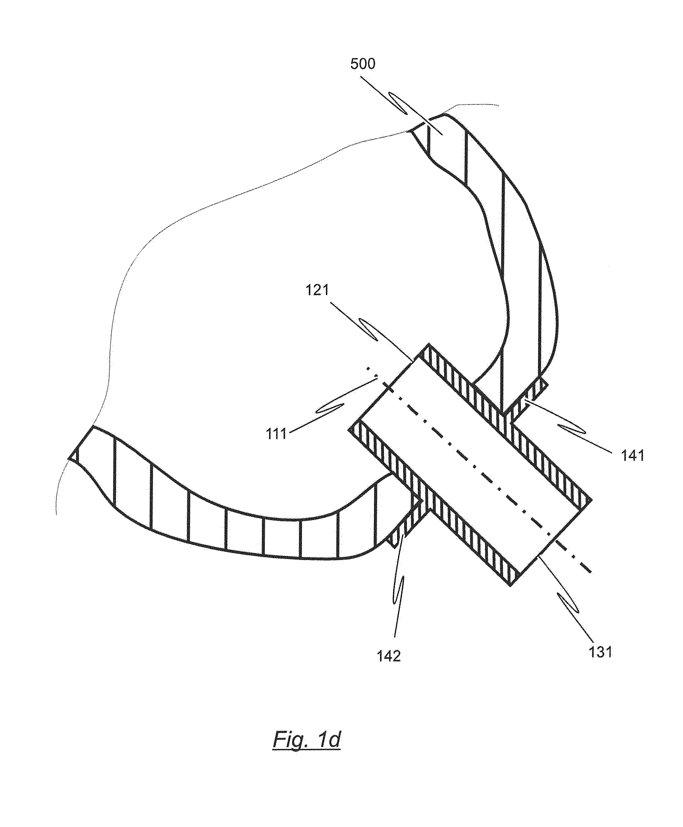

Fig. 1d shows a cross-sectional view of the access device 100 according to Fig. 1a to Fig. 1c, which is introduced into an opening of the wall 500 of the heart in the apical area. As depicted in Fig. 1d, the attachment means 141, 142 form contact with the surrounding tissue of the opening of the wall 500 of the heart into which the device is inserted. The inner annular lumen of the tubular access device 100 is adapted such that surgical instruments or a catheter system in which a prosthesis, e.g. a stent having a replacement heart valve affixed thereto to be implanted in the patient's body is accommodated, can be introduced.

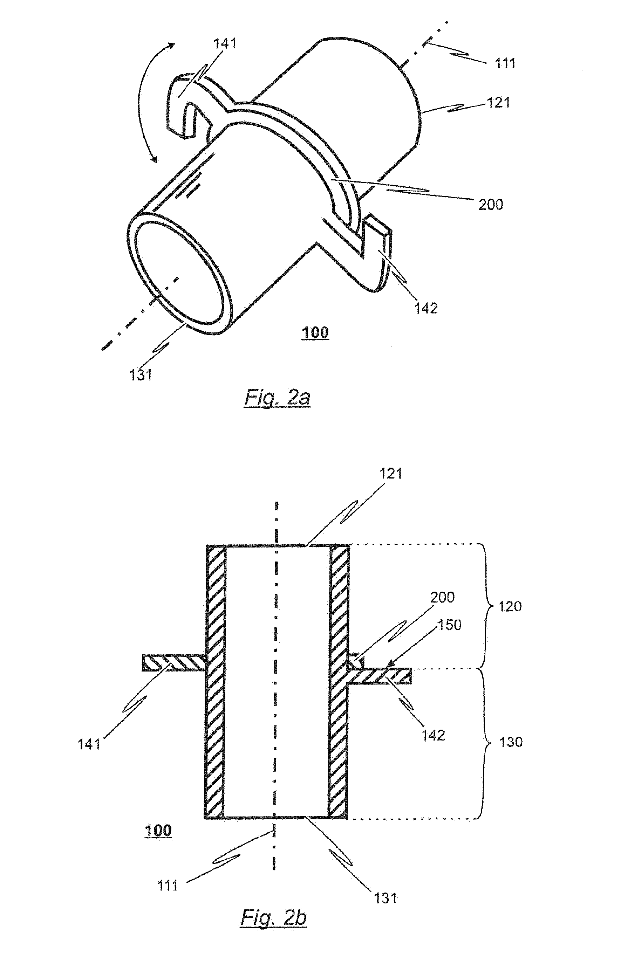

According to a preferred embodiment disclosed herein, one of the at least two attachment means 141, 142 of the access device 100 is rotatable relative to the other. An exemplary embodiment, wherein one of the at least two attachment means 141, 142 of the access device 100 is rotatable mounted on the exterior surface of the tubular element 110 of the access device 100 is shown in Fig. 2a and Fig. 2b. As depicted in a perspective view in Fig. 2a, one of two attachment means 141, 142 is an integral part of the tubular element 110 of the access device 100 or fixedly connected to the exterior surface of the tubular element 100, while the other attachment means 141 is an integral part of a ring 200 or fixedly connected to a ring 200, which is rotatable mounted on the exterior surface of the tubular element 110 of the access device 100.

Fig. 2b indicates, that in an exemplary embodiment the at least two attachment means 141, 142 are arranged, such that they are diametrically opposed in an initial position. The ring 200 with the attachment means 141 is preferably mounted on the exterior surface of the tubular element 110 of the access device 100 such that a rotation of the ring around the axis 111 of the access device 100 of about +/-30° can be realized. As shown in Fig. 2a, the attachment means 142 which is an integral part of the tubular element 110 or fixedly connected to the exterior surface of the tubular element 110 forms a stop 150 in axial direction for the ring 200. Therefore, when the ring is rotated around the axis 111 of the access device 100, the ring slides on the attachment means 142 which is fixedly connected to the exterior surface of the tubular element 100 as well as on the exterior surface of the tubular element 110. Thereby a guided rotational motion can be realized.

By providing an access device 100, wherein one of at least two attachment means 141, 142 is rotatable around the axis 3:11 of the access device 100, the handling during the fixation of the access device 100 to the wall of the heart by means of concentric purse-string sutures 900 is facilitated. Especially, bringing the attachment means 141, 142 in engagement with concentric purse-string sutures 900 surrounding the opening of the wall 500 of the heart is much easier when one of the at least two attachment means 141, 142 is rotatable. Furthermore, the stress applied to the tissue to which the attachment means 141, 142 are fixed is reduced, since potential rotational motions of the access device are not transmitted into the tissue, but compensated by the rotational motion of the attachment means 141, 142.

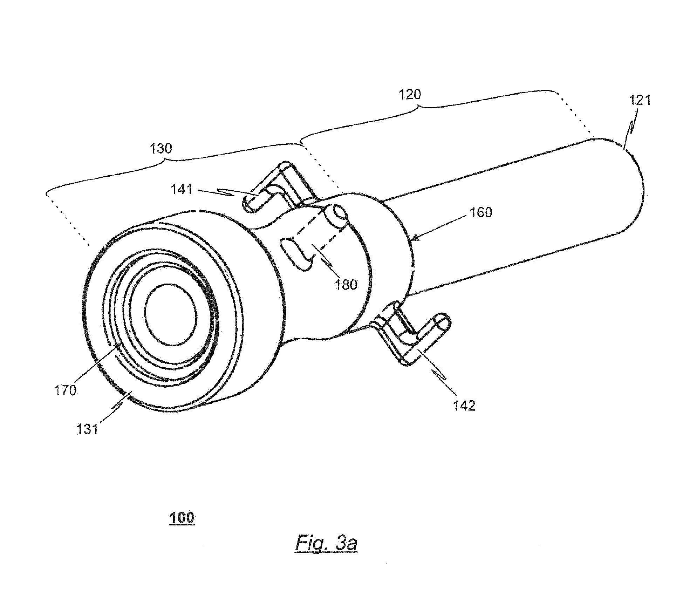

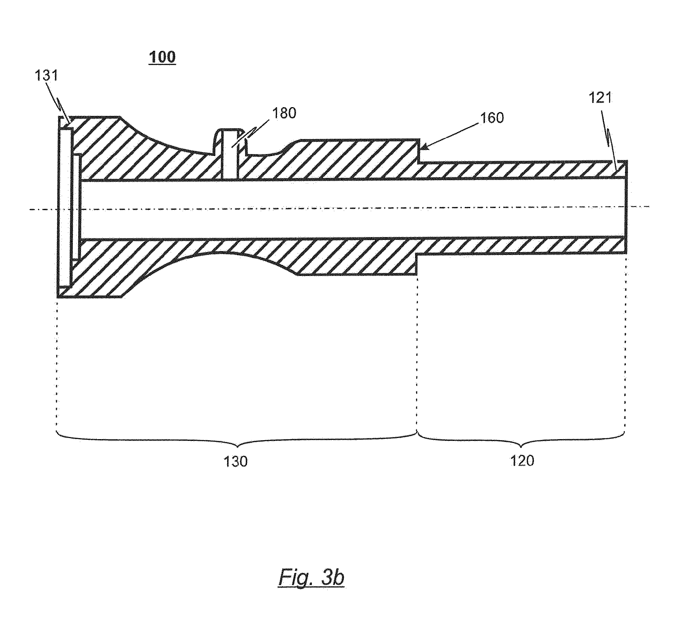

The following, referencing Fig. 3a and Fig. 3b, will describe the design of a further exemplary preferred embodiment of the access device of the present invention.

As depicted in Fig. 3a and Fig. 3b, an exemplary embodiment of the access device 100 comprises a tubular element 110 having a first portion 120 with a first outer diameter and a second portion 130 with a second outer diameter, wherein the first outer diameter is smaller than the second outer diameter such that a stop 160 is formed at the transition from first portion 120 to the second portion 130. Preferably, the stop 160 is formed by a surface which is substantially perpendicular to the axis 111 of the access device 100.

When the access device 100 is employed for the realization of a sealed passage during transapical surgery, the first portion 120 of the tubular element 110 having the smaller diameter is inserted into the opening in the wall 500 of the heart. Preferably, the outer diameter of the first portion 120 of the tubular element 110 is equal or slightly larger than the inner diameter of the opening in the wall 500 of the heart, such that a sealed and tight connection between the access device 100 and the surrounding tissue is established. The access device 100 is positioned in axial direction, such that the stop 160 forms contact with the outer surface of the wall 500 of the heart.

The stop 160 which is typically formed by a surface which is substantially perpendicular to the axis 111 of the tubular element 110 of the access device 100, on the one hand aids to stabilize the position of the access device 100 and on the other hand improves the sealing in order prevent significant blood loss and outflow of blood from the ventricle during the transapical procedure.

In detail, the stop 160 forming contact with the outer surface of the wall 500 of the heart is adapted, such that tilting of the access device 100 relative to the surface of the heart can be avoided. Thereby, the risk of tissue damage to the apical area of the heart is reduced, since tilting of the access device 100 would yield increased stress to the surrounding tissue of the opening in the wall 500 of the heart, into which the access device is inserted.

In the exemplary embodiment as depicted in Fig. 3a, at least two attachment means 141, 142 are connected to the second portion 130 of the tubular element 110. As shown in Fig. 3a, the at least two attachment means 141, 142 are preferably located just behind the stop 160 towards the proximal end 131 on the outer surface of the second portion 130 of the tubular element 110. The at least two attachment means 141, 142 are designed, such that they are in the same plane of the stop 160 or in a plane which is parallel to the plane represented by the stop 160. Preferably, said parallel plane is located within the second portion 1300 of the tubular element 110. With an access device 100 having attachment means 141, 142, which are in a plane which is parallel to the plane represented by the stop 160, the force transmitted to the wall 500 of the heart directed to the interior of the heart is further increased when the device 100 is fixed by means of purse string sutures 900.

All planes as described above are preferably perpendicular to the axis 111 of the access device 100.

The proximal end 131 of the access device 100, as shown in Fig. 3a and Fig. 3b, comprises an adapter 170 which is suited for sealably connecting a catheter system or a medical delivery system. Preferably, the second portion 130 of the tubular element 110 comprises a side port 180, which is suitable for introduction of medicaments or other fluids through the lumen of the tubular element 110 of the access device 100.

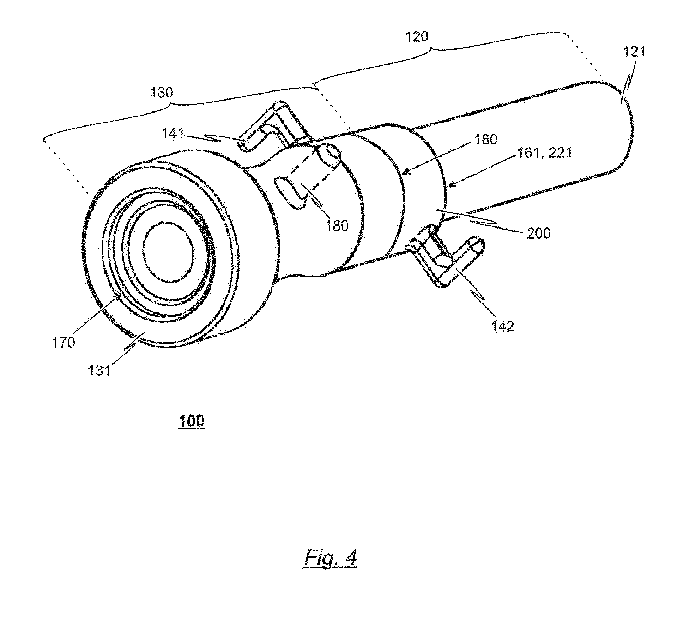

In another exemplary embodiment of the access device 100, as shown in Fig. 4, one of the at least two attachment means 141, 142 is an integral part of the second portion 130 of the tubular element 110 or fixedly connected to the second portion 130 of the tubular element 110 of the access device 100. The other of the at least two attachment means 141, 142 is an integral part of a ring 200 or fixedly connected to a ring 200, which is rotatable mounted on the exterior surface of the first portion 120 of the tubular element 110 of the access device 100.

In axial direction the ring 200 forms contact with the stop 160 and when rotated the ring slides on the exterior surface of the first portion 120 of the tubular element 110. Preferably, the ring 200 is mounted and adapted such that a rotation of the ring 200 around the axis 111 of the access device 100 of about +/-30° can be realized. In this exemplary embodiment, the ring 200 is adapted such that at the distal end 221 of the ring 200 forms a stop 161. The surface of the stop 161 is substantially perpendicular to the axis 111 of the tubular element 110 of the access device 100. When the access device 100 is inserted into the opening in the wall 500 of the heart in order to establish a sealed passage, the stop 161 represented by the distal end 221 of the ring 200 forms contact with the outer surface of the wall 500 of the heart.

Similarly as in the exemplary embodiment shown in Fig. 3a and 3b, the stop 161 aids to stabilize the position of the access device 100 and improves the sealing effect. Furthermore, the stop 161 represented by the distal end 221 of the ring 200, is adapted such that tilting of the access device 100 relative to the surface of the heart is hindered, when contact with the outer surface of the wall 500 of the heart is formed. Hence, the risk of tissue damage to the apical area of the heart is reduced, since tilting of the access device 100 would yield increased stress to the surrounding tissue of the opening in the wall of the heart, where the access device is positioned.

Although, not shown in Fig. 4, the at least two attachment means 141, 142 are preferably designed, such that they are in the same transversal plane. Preferably, said transversal plane is identical to the plane represented by the stop 161 or located somewhere behind the stop 161 towards the proximal end 131 of the access device 100.

The access device 100, as depicted in Fig. 4 and as outlined above, wherein one of at least two attachment means 141, 142 is rotatable around the axis 111 of the access device 100, facilitates the handling during the fixation of the access device 100 to the wall 500 of the heart by means of concentric purse-string sutures 900. Due to the fact that bringing the attachment means 141, 142 in engagement with concentric purse-string sutures 900 surrounding the opening of the wall 500 of the heart is much easier, when one of the at least two attachment means 141, 142 is rotatable, the time required for fixing the access device to the wall of the heart is reduced. Furthermore, the stress applied to the tissue of the heart to which the attachment means 141, 142 are fixed is reduced, since potential rotational motions of the access device 100 are not transmitted into the tissue, but compensated by the rotation of the attachment means 141, 142 around the axis 111 of the access device 100.

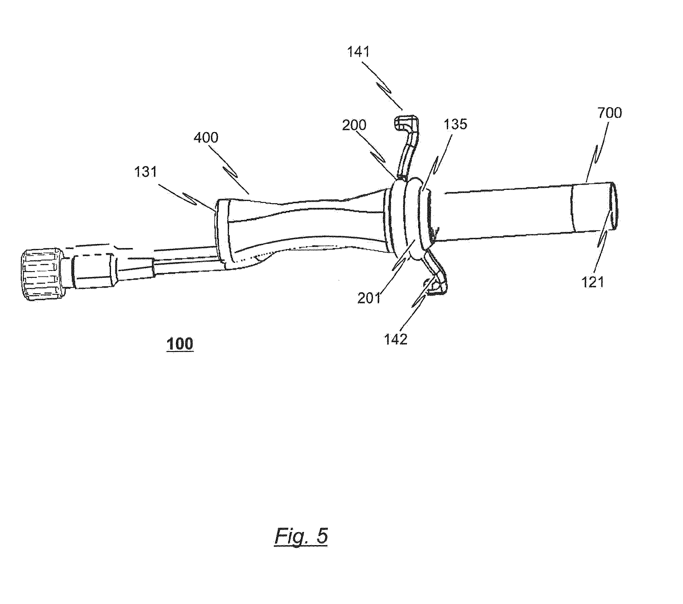

In another exemplary embodiment of the access device 100, as shown in Fig. 5, the access device 100 comprises two rings 200, 201 each with at least one attachment means 141, 142. The two rings 200, 201 comprising at least one attachment means 141, 142 can either be fixedly mounted to exterior surface of the tubular element 110 or such that at least one of the two rings 200, 201 is rotatable relative to the other ring around the axis 111 of the access device 100. The rings 200, 201 are adapted such that they can be fixed at a defined position onto the exterior surface of the tubular element by any kind of appropriate fixing means, e.g. a clip- or coupling-mechanism.

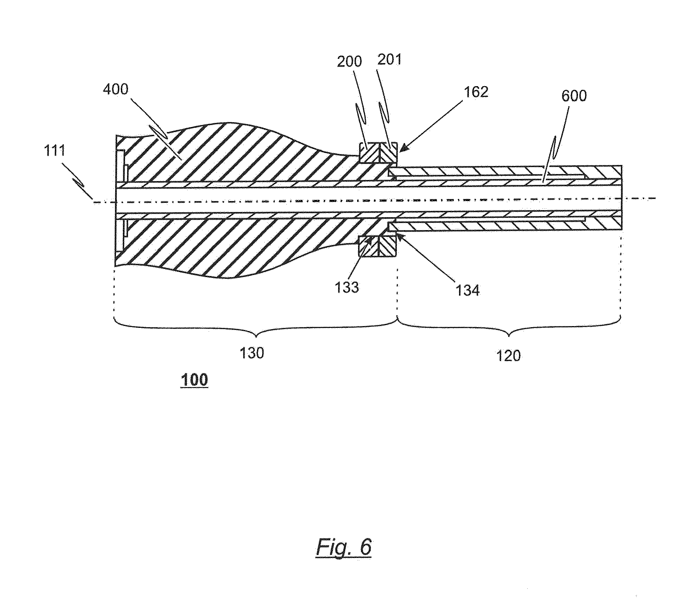

As shown in a cross-sectional view of a preferred embodiment in Fig. 6, the second portion 130 of the tubular element 110 has a recessed portion 133 at its distal end. Preferably, the two rings 200, 201 are mounted on the exterior surface of said recessed portion 133. The axial dimension of said recessed portion 133 is designed such that when the two rings 200, 201 are mounted the distal transversal end face 134 of the recessed portion 133 is in the same plane as the second ring 201 mounted on the recessed portion 133.

However, as indicated in Fig. 5, the recessed portion 133 can also be designed, such that when the two rings 200, 201 are mounted, a frustoconical distal end 135 of the recessed portion 133 protrudes. Preferably, the frustoconical distal end 135 of the recessed portion 133 is designed such that the smallest diameter is equal or slightly smaller than the opening in the wall 500 of the heart into which the device is introduced, while the largest diameter of the protruding frustoconical distal end 135 of the recessed portion 133 is slightly larger than the opening in the wall 500 of the heart. Thereby, when the access device 100 is inserted into the opening in the wall 500 of the heart and the distal end 135 of the recessed portion 133 forms contact with the wall 500, the surrounding tissue is under slight and equal tension around the circumference of the opening. Hence, providing an access device 100 as outlined above the sealing effect is further improved and blood outflow from the ventricle can be prevented, due to the fact that a stronger connection between the access device 100 and the surrounding tissue can be established.

As depicted in Fig. 5 and Fig. 6, the second portion of the tubular element may be designed to form an ergonomic handle 400.

In the exemplary embodiment according to Fig. 6, the second ring 201 is adapted such that at the distal end of the second ring 201 forms a stop 162. Preferably, said stop 162 has a surface which is substantially perpendicular to the axis 111 of the tubular element 110 of the access device 100.

When the access device 100 is introduced into the opening in the wall of the heart in order to establish a sealed passage, the stop 162 represented by the distal end of the second ring 201 forms contact with the outer surface of the wall 500 of the heart.

Similarly to the exemplary embodiment as shown in Fig. 4, the stop 162 aids stabilizing the position of the access device 100 while at the same time the sealing effect is improved. In detail, the stop 162 represented by the distal end of the second ring 201, is adapted such that tilting of the access device 100 relative to the surface of the heart can be avoided, when contact with the outer surface of the wall of the heart is formed. Hence, the risk of tissue damage in the apical area of the heart is reduced, since tilting of the access device 100 would yield increased stress to the surrounding tissue of the opening in the wall 500 of the heart, where the access device is positioned.

Although, not shown in Fig. 5, the at least two attachment means 141, 142 are preferably designed, such that they are in the same transversal plane. Preferably said transversal plane is identical to the plane represented by the stop 162 or located within the recessed portion 133 of the second portion 130 of the tubular element and parallel to the plane represented by the stop 162.

Similarly to the exemplary embodiments as shown in Fig. 2a and Fig. 2b as well as in Fig. 4, the embodiments of the access device 100 as depicted in Fig. 5 and Fig. 6, facilitate the handling during the fixation of the access device 100 to the wall 500 of the heart by means of concentric purse-string sutures 900. Due to the fact that bringing the attachment means 141, 142 in engagement with concentric purse-string sutures 900 surrounding the opening of the wall of the heart is much easier, the time required for fixing the access device 100 to the wall of the heart is be reduced. Furthermore, the stress applied to the tissue of the heart to which the attachment means 141, 142 are fixed is reduced, since potential rotational motions of the access device are not transmitted into the tissue, but compensated by the rotation of the attachment means 141, 142 around the axis 111 of the access device 100.

As shown in Fig. 6 the first portion 120 of the tubular element 110 of the access device100 may be a separate component, which is fixedly connected to the second portion 130 of the tubular element 110 by appropriate attachment means, e.g. by a clip- or coupling-mechanism. Thereby, different first tubular portions 120 are mountable onto the distal end of the second portion 130 of the access device 100. Hence, different first tubular portions 120 of the access device 100 having different length and/or different outer diameter can be employed. Therefore, the access device 100 can be individually adapted according to the size of the patient's heart as well as to specific surgical procedure requirements. Using an access device 100 with an elongated first tubular portion 120 might be advantageous for a procedure in which guiding of the catheter along the axis 111 of the access device 100 is needed.

Furthermore and as depicted in Fig. 6, the access device 100 may comprise an inner tube 600 extending along the axis 111 from the proximal end 131 to the distal end 121 of the access device 100. The inner annular lumen of the inner tube 600 of the access device 100 is adapted such that surgical instruments or a catheter system in which a prosthesis, e.g. a stent having a replacement heart valve affixed thereto to be implanted in the patient's body is accommodated, can be introduced.

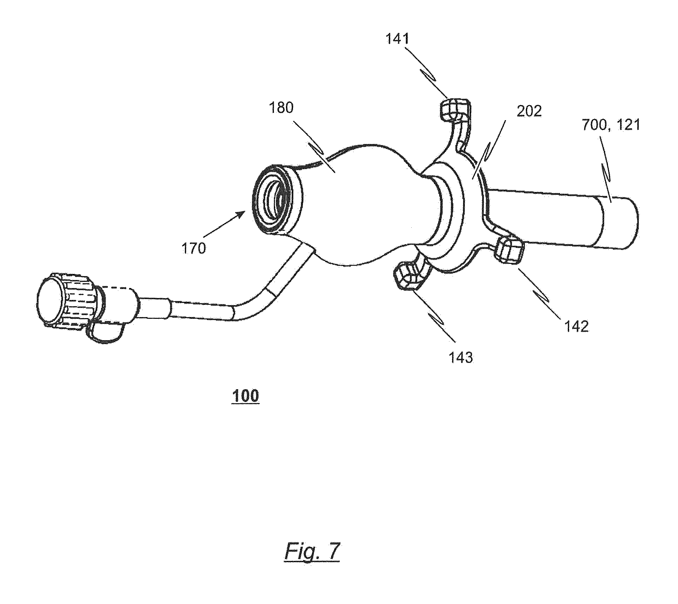

In an exemplary embodiment, as shown in Fig. 7, the access device 100 comprises a ring 202 with at least three attachment means 141, 142, 143, wherein the at least three attachment means 141, 142, 143 are distributed equally around the circumference of the ring 202, such that the spacing between two neighboring attachment means 141, 142, 143 is equal for all the at least three attachment means 141, 142, 143.

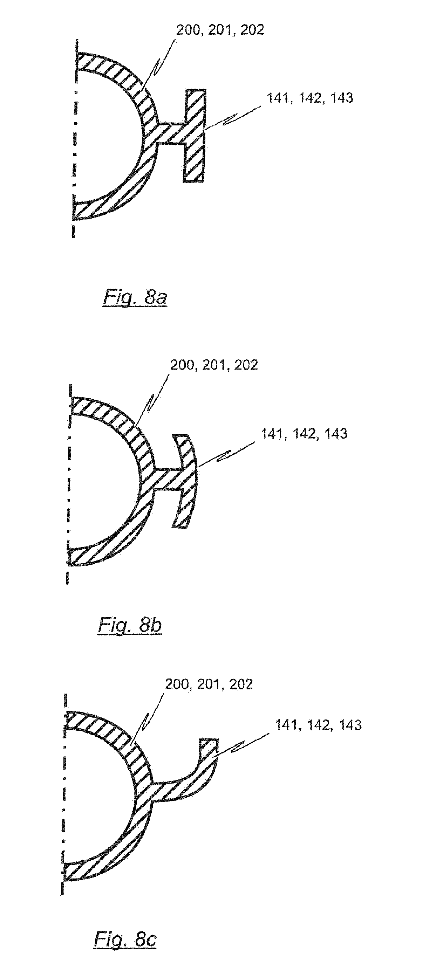

The various embodiments of the attachment means 141, 142, 143 will be described referencing Fig 8a to 8f.

Fig. 8a to Fig. 8c show horizontal cross-sectional views of a ring 200, 201, 202 with an attachment means 141, 142, 143 for visualizing a possible realizations of the attachment means 141, 142, 143: In Fig 8a the attachment means 141, 142, 143 is T-shaped; in Fig 8b the attachment means 141, 142, 143 has a concentric curved shape; and in Fig 8c the attachment means 141, 142, 143 has a quadrant- or L-shape.

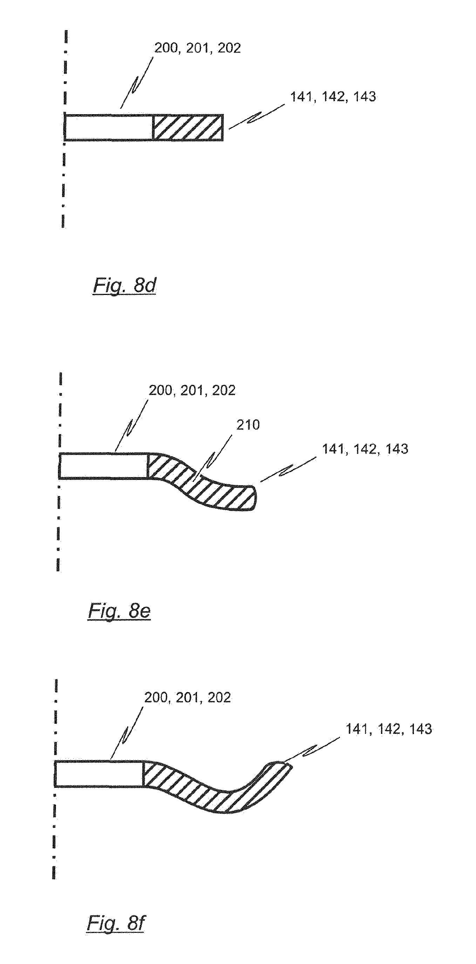

Fig. 8d to 8f axial illustrate cross-sectional views of a ring 200, 201, 202 with an attachment means 141, 142, 143 for visualizing possible realizations of the attachment means 141, 142, 143. Fig. 8d illustrates an embodiment of the attachment means 141, 142, 143 in which they are planar with the ring 200, 201, 202. Fig. 8e depicts an exemplary embodiment in which the attachment means 141, 142, 143 are connected to the ring 200, 201, 202 by a bended transition region 210 such that the attachment means 141, 142, 143 are in a different axial plane than the ring 200, 201, 202. Fig. 8f shows an embodiment in which the attachment means 141, 142, 143 has a hook-like shape in axial direction.

With an access device 100 having attachment means 141, 142, 143, which are located just behind the stop 160, 161, 162 towards the proximal end 131 of the access device 100 in a plane which is parallel to the plane represented by the stop 160, 161, 162, the force transmitted to the wall 500 of the heart directed to the interior of the heart is further increased when the device 100 is fixed by means of purse string sutures 900.

Although not shown in detail, any combinations of the embodiments shown in the horizontal cross-sectional views of Fig. 8a to Fig. 8c with the embodiments shown in the axial cross-sectional views of Fig. 8d to 8f can be realized and employed in the access device 100 according to the present invention.

Although not shown in detail, it is possible to equip the access device of the present invention with a telescope mechanism in order to adjust the length of the tubular first portion 120 of the access device 100, which protrudes into the interior of the heart when the device is inserted and fixed to the apical area of the heart.

According to a further aspect of access device of the present invention, the distal end 121 of the access device 100 may comprise a radiopaque tip 700 in order to visualize the position of the access device 100 in the operating field inside the heart by means of an appropriate imaging system. The imaging system may be used at any time or throughout the duration of the surgical procedure.

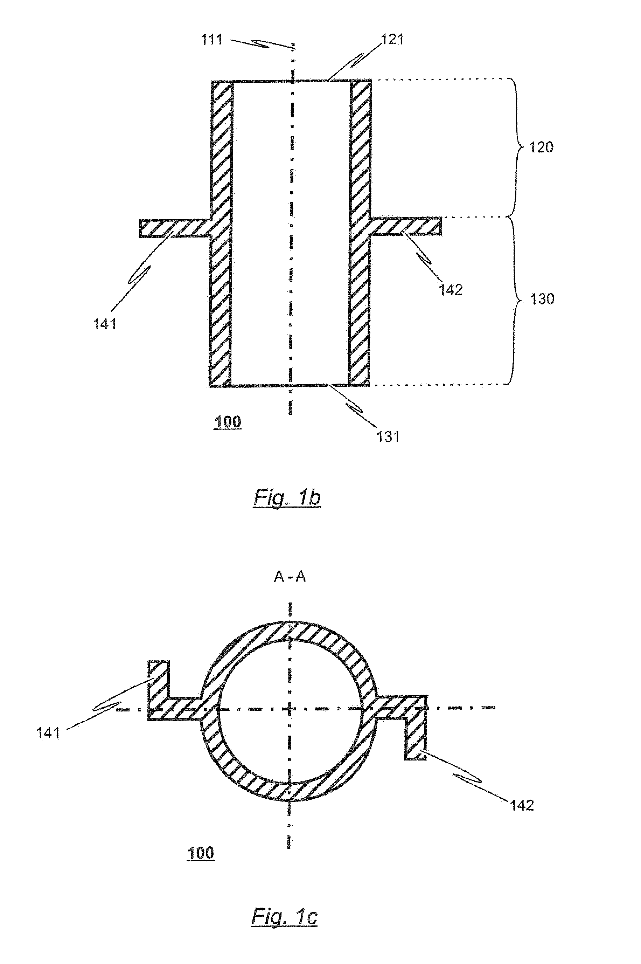

The various embodiments of the inventive access device 100 as illustrated in Fig. 1 to Fig. 8 may preferably be made of biocompatible material, such as stainless steel, Ti, Ti alloys, Ni alloys, Co alloys and biocompatible polymers.

A preferred method for fixing the access device 100 to the wall 500 of the heart, in particular to the apical area of the heart, shall comprise the following method steps:
i) providing an access device 100 in accordance with anyone of the previously described exemplary embodiments of the present invention;
ii) inserting the distal end 121 of access device 100 into the opening of the wall 500 of the heart; such that the attachment means 141, 142 form contact with the surrounding tissue of the opening of the wall 500 of the heart.
iii) fixing the access device 100 to the wall 500 of the heart, such that a stable sealed passage for communication with the interior of the heart is established, wherein the method step iii comprises the following steps:
   iii-a firstly the at least two attachment means 141, 142 are engaged with concentric purse-string sutures 900 surrounding the opening of the wall 500 of the heart;
   iii-b secondly the ends 901 of the purse-string sutures 900 are fed through a tourniquet 920 and upon pulling the ends 901 of purse-string sutures 900 a force is transmitted to the wall 500 of the heart directed to the interior of the heart via the attachment means 141, 142, such that a stable and sealed connection is established between the wall 500 of the heart and the access device 100; and
   iii-c thirdly the ends 901 of the purse-string sutures 900 are immobilized at the proximal end 931 of the tourniquet 920 such that the distal end 921 of the tourniquet 920 is pressed against the wall 500 of the heart and/or against one of the at least two attachment means 141, 142, such that the established connection between the wall of the heart and the access device 100 is sustained.

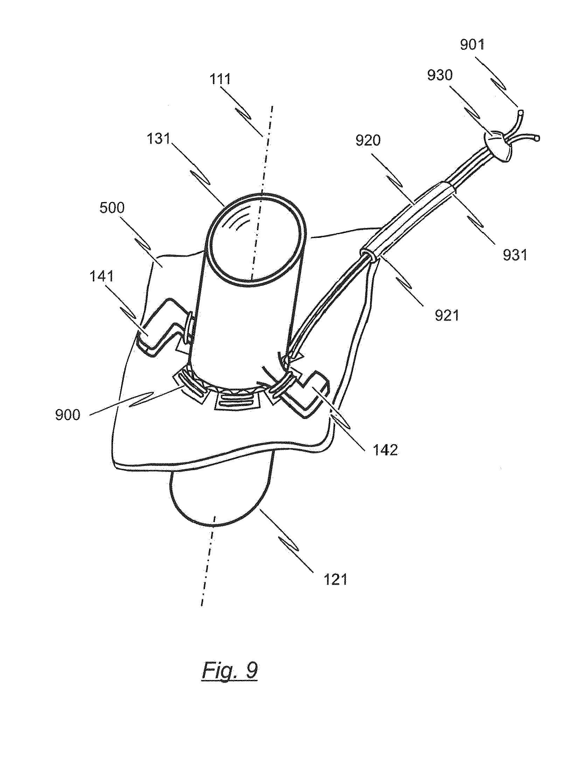

Exemplary, in Fig. 9 an access device 100 as depicted in Fig. 1a to Fig. 1d is used to illustrate the principle how the access device 100 is fixed to the apical area of the heart by means of concentric purse-string sutures 900. However, the principle and method as outlined above is applicable to all possible embodiments of the inventive access device as disclosed herein.

In particular the access device in combination with the purse-string 900 and the tourniquet 920 forms part of the invention as a set in its un-deployed form or deployed on the heart.

As illustrated in Fig. 9, the radial outwardly extending attachment means 141, 142 of the access device 100 are brought into engagement with concentric purse-string sutures 900 surrounding the opening of the wall 500 of the heart. After the access device 100 is inserted into the heart, the purse strings 900 and tourniquets 920 are used to secure the access device 100 to the heart. Once the purse strings 900 and tourniquets 920 are tightened, the ends 901 of the purse-string sutures 900 are immobilized by appropriate fastening means 930.

The disclosed solution is not limited to the embodiments described with reference to the accompanying drawings. Also just as conceivable in fact are combinations of the individual features as specifically described.

## Claims

1. Access device (100) for cardiac stent delivery, in particular for delivery of a expandable stent having a replacement heart valve affixed thereto to a heart through a wall of the heart, comprising a tubular element (110) adapted to be sealably inserted into an opening of a wall of the heart for communication with the interior of the heart, wherein the access device (100) includes at least two radial outward extending attachment means (141, 142) adapted to engage with concentric purse-string sutures (900) surrounding the opening of the wall (500) of the heart for fixing the access device (100) to the wall (500) of the heart.

2. Access device (100) according to claim 1,
wherein the access device (100) is in engagement with purse-string sutures (900), wherein the ends (901) of the purse-string sutures (900) are fed through a tourniquet (920), which are immobilized at a proximal end (931) of the tourniquet (920), such that a distal end (921) of the tourniquet is pressed against the wall (500) of the heart and/or against one of the at least two attachment means (141, 142), such that an established connection between the wall of the heart and the access device (100) is maintained in a sustained manner.

3. The access device (100) according to claim 1 or 2,
wherein the tubular element (110) comprises a side port (180) extending at an angle from the tubular element (110), wherein the side port (180) is adapted for introduction of medicaments or other fluids through the lumen of the tubular element (110).

4. The access device (100) according to claim 1 to 3,
wherein the tubular element (110) comprises a first portion (120) having a first outer diameter and a second portion (130) having a second outer diameter, wherein the first outer diameter is smaller than the second outer diameter such that a stop (150) is formed having a surface which is substantially perpendicular to the axis (111) of the tubular element (110), wherein said stop (150) is adapted to form a substantially sealed contact with the outer surface of the wall (500) of the heart, and wherein the second portion (130) of the tubular element (110) comprises said at least two attachment means (141, 142).

5. The access device (100) according to any one of claims 1 to 4,
wherein the length of tubular element (110) can be adjusted along the axis (111) of the tubular element (110).

6. The access device (100) according to any one of claims 1 to 5,
wherein the distal end (121) of the access device (100) comprises a radiopaque tip (700).

7. The access device (100) according to any one of claims 1 to 6,
wherein one of the at least two attachment means (141, 142) is rotatable mounted on the exterior surface of the tubular element (110), such that at least one of the at least two attachment means (141, 142) is rotatable around the axis (111) of the tubular element (110).

8. The access device (100) according to any one of claims 1 to 7,
wherein one of the at least two attachment means (141, 142) is an integral part of a ring (200) or fixedly connected to a ring (200) which is rotatable mounted on the exterior surface of the tubular element (110), such that the ring (200) is rotatable around the axis (111) of the tubular element (110).

9. The access device (100) according to claim 8,
wherein the ring (200) is mounted on the exterior surface of the tubular element (110), such that the ring (200) slides on a surface of one of the at least two attachment means (141, 142) which faces towards the distal end (131) of the access device (100).

10. The access device (100) according to any one of claims 1 to 9,
wherein one of the at least two attachment means (141, 142) is diametrically opposed to the other of the at least two attachment means (141, 142).

11. The access device (100) according to any one of claims 1 to 10,
wherein at least one of the at least two attachment means (141, 142) is rotatable about +/-30° relative to the diametrically opposed attachment means (141, 142).

12. The access device (100) according any one of claims 1 to 11,
wherein said attachment means (141, 142) are L-shaped, T-shaped, quadrant-shaped or semicircular-shaped hooks.

13. The access device (100) according any one of claims 1 to 12,
wherein the access device being preferably made of biocompatible material, said biocompatible material being preferably selected from the group consisting of stainless steel, Ti, Ti alloys, Ni alloys, Co alloys and polymers, preferably biocompatible.

14. The access device (100) according any one of claims 1 to 13,
wherein the second portion (130) of the tubular element (110) comprises a handle (400).

15. Method for fixing an access device (100) to the wall (500) of the heart, wherein the method comprises the following steps:
i) providing an access device (100) according to any of claims 1 to 12;
ii) inserting the access device (100) into the opening of the wall (500) of the heart; and
iii) fixing the access device (100) to the wall (500) of the heart,
wherein in procedural step iii) comprises the following steps
iii-a) the at least two attachment means (141, 142) are engaged with concentric purse-string sutures (900) surrounding the opening of the wall (500) of the heart;
iii-b) the ends (901) of the purse-string sutures are fed through a tourniquet (920) and upon pulling the ends (901) of purse-string sutures (900) a force is transmitted to the wall (500) of the heart directed to the interior of the heart via the attachment means (141, 142), such that a stable and sealed connection is established between the wall (500) of the heart and the access device (100); and
iii-c) the ends (901) of the purse-string sutures (900) are immobilized at the proximal end (931) of the tourniquet (920) such that the distal end (921) of the tourniquet is pressed against the wall (500) of the heart and/or against one of the at least two attachment means (141, 142), such that the established connection between the wall of the heart and the access device (100) is sustained.
